# EUROPEAN PATENT APPLICATION

(11) **EP 3 769 677 A1**
(43) Date of publication of application: **27.01.2021**
(21) Application number: 19770495.0
(22) Date of filing: 08.02.2019
(51) Int. Cl.: A61B 5/1455, A61B 5/02, A61B 5/0245, A61B 10/00

(54) **BIOLOGICAL INFORMATION MEASUREMENT DEVICE**

(30) Priority: 20.03.2018 JP 2018052385
(71) Applicant: Dynamic Brain Lab, LLC., Tokyo 162-0842 (JP)
(72) Inventor: VALENZI Stefano, Tokyo 135-0044 (JP); JURICA Peter, Wako-shi, Saitama 351-0104 (JP)
(74) Representative: Cabinet Nuss
(86) International application number: PCT/JP2019/004641
(87) International publication number: WO 2019/181267

(57) **Abstract**

A biological information measurement device includes: a first sensor module (21) which includes one or multiple light-emitting elements (30, 31, and 32) emitting light and one or multiple light-receiving elements (40 and 41) receiving light emitted from the light-emitting elements (30, 31, and 32) and outputting a signal of an intensity corresponding to an amount of light to be received; and a second sensor module (22) which includes one or multiple light-emitting elements (33) emitting light, wherein at least one light-receiving element of the first sensor module (21) receives light emitted from at least one light-emitting element of the first sensor module (21) and reflected by an organism and receives light which has been emitted from at least one light-emitting element of the second sensor module (22) and which has been transmitted through an organism.

## Description

### [Technical Field]

The present invention relates to a biological information measurement device. Particularly, the present invention relates to an optical biological information measurement device.

### [Background Art]

In the related art, as optical non-invasive biological information measurement devices, blood glucose level sensors, pulse oximeters, and the like are known. An optical blood glucose level sensor irradiates biological tissues with near-infrared light, measures an absorbance spectrum by guiding diffuse reflected light from biological tissues to a light-receiving means, and measures a glucose concentration in the blood of the organism in a non-invasive manner (for example, refer to Patent Literature 1). A pulse oximeter measures the oxygen saturation in the blood of biological tissues in a non-invasive manner (for example, refer to Patent Literature 2). The principle is to measure the oxygen saturation in the blood of an organism by emitting light having different wavelengths to the biological tissues using light-emitting elements and detecting the absorbance of each transmitted light using a difference in absorbance of light by oxy-hemoglobin and reduced hemoglobin for light having a predetermined wavelength.

### [Citation List]

### [Patent Literature]

[Patent Literature 1]
   Japanese Patent Laid-Open No. 2005-080710
[Patent Literature 2]
   Japanese Patent Laid-Open No. H06-098881

### [Summary of Invention]

### [Technical Problem]

However, the biological information measurement devices in the related as described in Patent Literature 1 and 2 acquire biological information by detecting only one of transmitted light transmitted through an organism and reflected light reflected by the organism using only one portion of the organism as an object on which measurement is performed.

For this reason, the biological information which is acquired is strongly affected by differences between portions of an organism and there is a problem that a measurement value may vary each time measurement is performed and it is difficult to obtain biological information with high accuracy.

An object of the present invention is to provide a biological information measurement device in which biological information can be obtained with high accuracy through a simple configuration.

In order to achieve the above object, a biological information measurement device of an aspect of the present invention includes: a first sensor module which includes one or multiple light-emitting elements emitting light and one or multiple light-receiving elements receiving light emitted from the light-emitting elements and outputting a signal of an intensity corresponding to an amount of light received; a second sensor module which includes one or multiple light-emitting elements emitting light; and a signal processing unit in which signal processing is performed on an output signal from the light-receiving elements to generate biological information, wherein at least one light-receiving element of the first sensor module receives light emitted from at least one light-emitting element of the first sensor module and reflected by an organism and receives light which has been emitted from at least one light-emitting element of the second sensor module and which has been transmitted through an organism.

Also, in order to achieve the above object, a biological information measurement device of an aspect of the present invention includes: a first sensor module which includes a first light-emitting element emitting light having a first wavelength; a second sensor module which includes a second light-emitting element emitting light having a second wavelength; a third sensor module which includes a third light-emitting element emitting light having a third wavelength; a fourth sensor module which includes a fourth light-emitting element emitting light having a fourth wavelength; a fifth sensor module which includes a first light-receiving element receiving light having the first wavelength and the second wavelength emitted from the first light-emitting element and the second light-emitting element and reflected by an organism and outputting a signal; a sixth sensor module which includes a second light-receiving element receiving light having the second wavelength and the third wavelength emitted from the second light-emitting element and the third light-emitting element and reflected by an organism and outputting a signal; and a signal processing unit in which signal processing is performed on output signals from the first light-receiving element and the second light-receiving element to generate biological information, wherein the first light-receiving element and/or the second light-receiving element receives light having the fourth wavelength emitted from the fourth light-emitting element and transmitted through an organism.

Also, in order to achieve the above object, a biological information measurement device of an aspect of the present invention includes: a first sensor module which includes a first light-emitting element emitting light having a first wavelength; a second sensor module which includes a second light-emitting element emitting light having a second wavelength; a third sensor module which includes a third light-emitting element emitting light having a third wavelength; a fourth sensor module which includes a fourth light-emitting element emitting light having a fourth wavelength; a fifth sensor module which includes a first light-receiving element receiving light having the first wavelength, the second wavelength, and the third wavelength emitted from the first light-emitting element, the second light-emitting element, and the third light-emitting element and reflected by an organism and outputting a signal; a sixth sensor module which includes a second light-receiving element receiving light having the fourth wavelength emitted from the fourth light-emitting element and reflected by an organism and outputting a signal; and a signal processing unit in which signal processing is performed on output signals from the first light-receiving element and the second light-receiving element to generate biological information, wherein the first light-receiving element receives light having the fourth wavelength emitted from the fourth light-emitting element and transmitted through an organism, and the second light-receiving element receives light having the first wavelength and/or the second wavelength and/or the third wavelength emitted from the first light-emitting element and/or the second light-emitting element and/or the third light-emitting element and transmitted through an organism.

### [Advantageous Effects of Invention]

According to the present invention, the present invention can provide a biological information measurement device in which biological information can be obtained with high accuracy through a simple configuration.

### [Brief Description of Drawings]

Fig. 1 is a diagram illustrating an example of a configuration of a sensor module in a biological information measurement device according to a first embodiment.
Fig. 2 is a diagram illustrating an example of a configuration of a sensor module in a biological information measurement device according to a second embodiment.
Fig. 3 is a diagram illustrating an example of a configuration of a sensor module in a biological information measurement device according to third embodiment.
Fig. 4 is a diagram illustrating an example of a configuration of a sensor module in a biological information measurement device according to a modified example of the first embodiment.
Fig. 5 is a diagram illustrating an example of a configuration of a sensor module in a biological information measurement device according to a modified example of the second embodiment.
Fig. 6 is a diagram illustrating an example of a configuration of a sensor module in a biological information measurement device according to a modified example of the third embodiment.
Fig. 7 is a diagram illustrating an example of a functional configuration of the biological information measurement device according to the first to third embodiments and the modified examples thereof.
Fig. 8 is a diagram illustrating an example of a functional configuration of a biological information measurement device according to a fourth embodiment.
Fig. 9 is a schematic diagram of an example of a first outer form of the biological information measurement device according to the fourth embodiment.
Fig. 10 is a schematic cross-sectional view of a cross section of an example of the first outer form of the biological information measurement device according to the fourth embodiment.
Fig. 11 is a schematic diagram of an example of a second outer form of the biological information measurement device according to the fourth embodiment.
Fig. 12 is a schematic diagram of an example of a third outer form of the biological information measurement device according to the fourth embodiment.
Fig. 13 is a schematic diagram of an example of a fourth outer form of the biological information measurement device according to the fourth embodiment.
Fig. 14 is a schematic diagram of an example of a fifth outer form of the biological information measurement device according to the fourth embodiment.
Fig. 15 is a schematic diagram of an example of the biological information measurement device according to the fourth embodiment attached to an external device.
Fig. 16 is a schematic diagram of a first band section configured to install and fix the biological information measurement device according to the fourth embodiment on biological tissues.
Fig. 17 is a schematic diagram of a second band section configured to install and fix the biological information measurement device according to the fourth embodiment on biological tissues.
Fig. 18 is a diagram for explaining an example of a system in which the biological information measurement device according to the fourth embodiment is used.

### [Description of Embodiments]

Various embodiments of a biological information measurement device according to the present invention will be described in detail below with reference to the drawings. In the following description made with reference to the drawings, the same or similar constituent elements will be denoted by the same or similar reference numerals. However, the drawings are schematic and dimensional ratios of constituent elements may be different from actual ratios. Furthermore, it is needless to say that portions having different dimensional ratios and the like are included in the drawings.

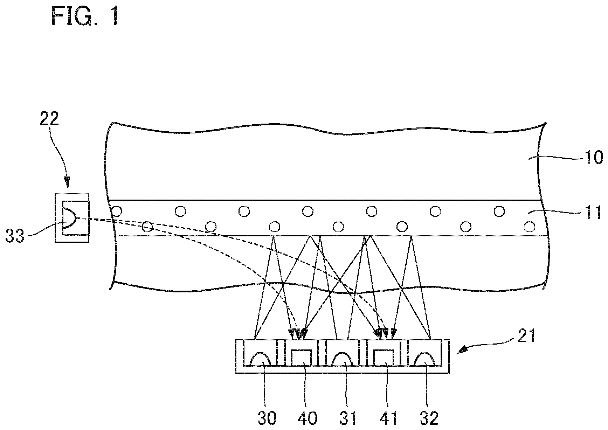

Figs. 1 to 3 are diagrams illustrating an example of a configuration of a sensor module in a biological information measurement device according to first to third embodiments. A biological information measurement device 100 according to the first to third embodiments includes two or more different sensor modules 20 and can measure two or more different portions of an organism 10. Thus, it is possible to reduce an influence of a difference between portions of the organism 10 and it is possible to obtain biological information with high accuracy. Furthermore, at least one light-receiving element of the sensor modules 20 receives the reflected light from each of light-emitting elements in the same sensor module and receives the transmitted light from each of light-emitting elements in different sensor modules. Thus, it is possible to detect both of the reflected light and the transmitted light with a simple configuration and it is possible to obtain biological information with high accuracy.

Here, the light-emitting element is an element which emits light to an organism and is a light emitting diode (LED) or the like. In this embodiment, an LED capable of emitting light including visible light to infrared light (for example, light having a wavelength of 0.36 µm to 2.5 µm) having subcutaneous transparency is used. The light-receiving element is an element which receives light which has passed through and been reflected by an organism and outputs an electrical signal corresponding to an amount of light received and is a photodiode or the like. In this embodiment, a photodiode is used as the light-receiving element.

The sensor modules 20 illustrated in Figs. 1 to 3 include the first sensor module 21 and the second sensor module 22. The first sensor module 21 includes N (N is greater than or equal to 3) LEDs and 1 or more and N-1 or less photodiodes. The second sensor module 22 includes one or multiple photodiodes.

### <First embodiment>

Fig. 1 is the diagram illustrating the example of the configuration of the sensor module in the biological information measurement device according to the first embodiment. The first sensor module 21 illustrated in Fig. 1 includes three LEDs and two photodiodes. For example, the LEDs of the first sensor module 21 are constituted of an LED 30 which emits infrared light having a wavelength greater than or equal to 1000 nm, an LED 31 which emits infrared light having a wavelength less than or equal to 1000 nm, and an LED 32 which emits visible light having a wavelength greater than or equal to 390 nm. The photodiodes of the first sensor module 21 are constituted of two photodiodes 40 and 41 in which light having a wavelength emitted by the LEDs 30, 31, and 32 of the first sensor module 21 and an LED 33 of the second sensor module 22 can be detected.

The second sensor module 22 includes one LED. For example, the LED of the second sensor module 22 is constituted of the LED 33 which emits infrared light having a wavelength greater than or equal to 1000 nm.

As shown in Fig. 1, a photodiode 40 of the first sensor module 21 receives light emitted from at least one LED of the LEDs 30, 31, and 32 of the first sensor module and reflected by an organism and receives light emitted from the LED 33 of the second sensor module 22 and transmitted through an organism. As in the photodiode 40, a photodiode 41 also receives light emitted from at least one LED of the LEDs 30, 31, and 32 of the first sensor module and reflected by an organism and receives light emitted from the LED 33 of the second sensor module 22 and transmitted through an organism.

Here, it is known that conditions appropriate for detecting an object to be detected, a region to be detected, a distance to be detected, and the like are different in both detection forms for the reflected light and the transmitted light. In the biological information measurement device 100 of the present invention, both of the reflected light and the transmitted light are detected. Thus, it is possible to calculate biological information by selecting the form of the conditions appropriate for detection, it is possible to calculate an average value of measurement values of both of the detection forms, and it is possible to acquire biological information with high accuracy. Furthermore, it is known that the measurement values of the biological information measurement devices in the related art may significantly differ in some cases depending on a portion to be measured due to an influence of pigmentation of an organism. Since the biological information measurement device of the present invention performs measurement on two different portions, it is possible to mitigate differences between portions and obtain biological information with high accuracy.

The first sensor module 21 has a configuration in which one LED is omitted as compared with the sensor modules in the related art as described in Patent Literature 1 and 2. It is desirable that the photodiode 40 of the first sensor module 21 receive light emitted from the two neighboring LEDs 30 and 31 and reflected by an organism. As in the photodiode 40, it is desirable that the photodiode 41 receive light emitted from the two neighboring LEDs 31 and 32 and reflected by an organism. That is to say, the two common photodiodes 40 and 41 which are adjacent to the LEDs receive the light emitted from the LEDs 30, 31, and 32 and reflected by the organism 10. With this configuration, an installation space required for the LED in the related art, a voltage loss due to a drive circuit, and the like are eliminated and it is possible to achieve a decrease in size of a device and saving of electric power.

Although a description has been provided of an example in which the two photodiodes 40 and 41 of the first sensor module 21 both receive the transmitted light from the LED 33 of the second sensor module 22, the present invention is not limited thereto. In addition, as long as at least one photodiode of the two photodiodes 40 and 41 of the first sensor module 21 can receive the transmitted light from the LED 33 of the second sensor module 22, the configuration may be adopted. The same applies to the following embodiments.

Also, any positional relationship between the first sensor module 21 and the second sensor module 22 is possible as long as the photodiodes 40 and 41 of the first sensor module 21 can detect the transmitted light emitted from the LED 33 of the second sensor module 22 and transmitted through an organism. Any positional relationship between the first sensor module 21 and the second sensor module 22 is possible as long as the positional relationship can be appropriately selected in accordance with a feature to be measured and a configuration of a device in which a sensor module is accommodated, and the present invention is not particularly limited. The same applies to the following embodiments.

### <Second embodiment>

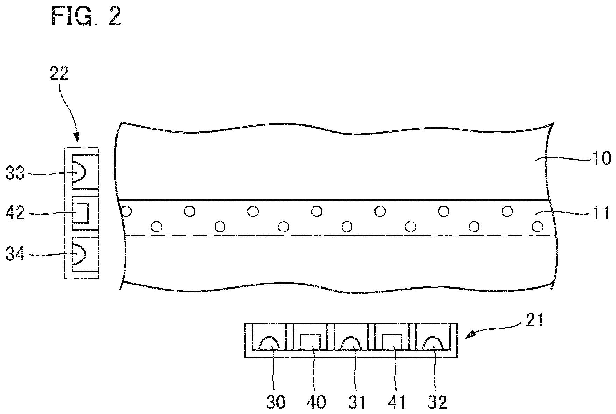

Fig. 2 is a diagram illustrating an example of a configuration of a sensor module in a biological information measurement device according to a second embodiment. A sensor module 20 in the second embodiment is different from the sensor module 20 in the first embodiment in that one or multiple LEDs are further provided in a second sensor module 22. Since the other configurations have the same configuration as the sensor module 20 in the first embodiment, constituent elements of the second embodiment that are the same as those of the first embodiment will be denoted by the same reference numerals as the first embodiment, a description thereof will be omitted, and different constituent elements will be described.

The second sensor module 22 illustrated in Fig. 2 includes two LEDs 33 and 34 and one photodiode 42. For example, LEDs of the second sensor module 22 are constituted of an LED 33 which emits infrared light having a wavelength greater than or equal to 1000 nm and an LED 34 which emits visible light having a wavelength greater than or equal to 390 nm. A photodiode of the second sensor module 22 is constituted of one photodiode 42 in which light having a wavelength emitted by LEDs 30, 31, and 32 of the first sensor module 21 and LEDs 33 and 34 of the second sensor module 22 can be detected.

It is desirable that the photodiodes 40 and 41 of the first sensor module 21 receive light emitted from the neighboring LEDs 30, 31, and 32 and reflected by an organism 10 and receive light emitted from at least one LED of the LEDs 33 and 34 of the second sensor module 22 and transmitted through the organism 10. The photodiode 42 of the second sensor module 22 receives light emitted from at least one LED of the LEDs 30, 31, and 32 of the first sensor module 21 and transmitted through the organism 10 and receives light emitted from the LEDs 33 and 34 of the second sensor module 22 and reflected by the organism 10.

Since the biological information measurement device in the second embodiment includes the photodiode 42 added to the second sensor module 22, in contrast to the biological information measurement device in the first embodiment, it is possible to acquire multiple pieces of biological information with higher accuracy.

### <Third embodiment>

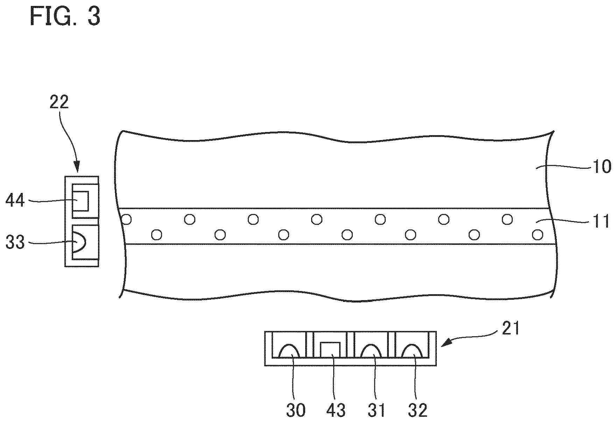

Fig. 3 is a diagram illustrating an example of a configuration of a sensor module in a biological information measurement device according to a third embodiment. A sensor module 20 in the third embodiment is different from the sensor module in the first embodiment in that one photodiode of the first sensor module 21 in the first embodiment is omitted and one LED is added to the second sensor module 22 in the first embodiment.

A first sensor module 21 illustrated in Fig. 3 includes three LEDs 30, 31, and 32 and one photodiode 43. For example, the LEDs of the first sensor module 21 are constituted of an LED 30 which emits infrared light having a wavelength greater than or equal to 1000 nm, an LED 31 which emits infrared light having a wavelength less than or equal to 1000 nm, and an LED 32 which emits visible light having a wavelength greater than or equal to 390 nm. A photodiode of the first sensor module 21 is constituted of one photodiode 43 in which light having a wavelength emitted by the LEDs 30, 31, and 32 of the first sensor module 21 and an LED 33 of the second sensor module 22 can be detected.

The second sensor module 22 illustrated in Fig. 3 includes one LED 33 and one photodiode 44. For example, an LED of the second sensor module 22 is constituted of the LED 33 which emits infrared light having a wavelength greater than and equal to 1000 nm. A photodiode of the second sensor module 22 is constituted of one photodiode 44 in which light having a wavelength emitted by the LEDs 30, 31, and 32 of the first sensor module 21 and the LED 33 of the second sensor module 22 can be detected.

It is desirable that the photodiode 43 of the first sensor module 21 receive light emitted from the LEDs 30, 31, and 32 of the first sensor module 21 and reflected by the organism 10 and receive light emitted from the LED 33 of the second sensor module 22 and transmitted through an organism 10. It is desirable that the photodiode 44 of the second sensor module 22 receive light emitted from at least one LED of the LEDs 30, 31, and 32 of the first sensor module 21 and transmitted through the organism 10 and receive light emitted from the LED 33 of the second sensor module 22 and reflected by the organism 10. With this configuration, the biological information measurement device in the third embodiment has the same effects as those of the first and second embodiments.

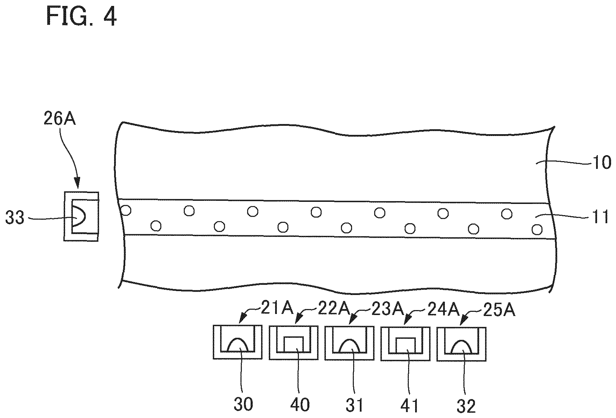

Figs. 4 to 6 illustrate examples of configurations of sensor modules according to modified examples of the first to third embodiments. A sensor module 20A according to the modified examples of the first to third embodiments is different from the sensor module 20 of the first to third embodiments in that the sensor module 20A includes only any one of an LED and a photodiode in one sensor module and a positional relationship between the LED and the photodiode is not fixed. With this configuration, a distance and an angle between the LED and the photodiode can be easily adjusted to obtain optimum detection conditions. An optical path through which light passes through an organism 10 changes in accordance with a wavelength of the light. A case in which the scattering and absorption of light is large and an amount of light reaching a photodiode is small in accordance with the organism 10 so that biological information cannot be calculated may be present in some cases. In such a case, the sensor module 20A in the modified examples in which the distance and the angle between the LED and the photodiode can be easily adjusted to obtain optimum detection conditions becomes useful. The other points, for example, combinations of LEDs and photodiodes, and the like are the same as those of the sensor modules in the first to third embodiments. For this reason, only the configuration of the sensor module 20A according to the modified examples will be described.

### <Modified example of first embodiment>

A sensor module 20A according to a modified example of the first embodiment illustrated in Fig. 4 includes first to sixth sensor modules 21A, 22A, 23A, 24A, 25A, and 26A. LEDs 30, 31, and 32 or photodiodes 40 and 41 of the first to fifth sensor modules 21A, 22A, 23A, 24A, and 25A according to the modified example illustrated in Fig. 4 correspond to the LEDs 30, 31, and 32 or the photodiodes 40 and 41 of the first sensor module 21 in the first embodiment and the LED 33 of the sixth sensor module 26A according to the modified example corresponds to the LED 33 of the second sensor module 22 in the first embodiment.

### <Modified example of second embodiment>

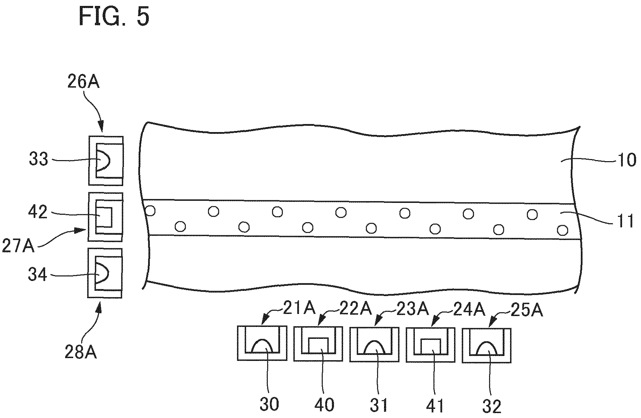

A sensor module 20A according to a modified example of the second embodiment illustrated in Fig. 5 includes first to eighth sensor modules 21A, 22A, 23A, 24A, 25A, 26A, 27A, and 28A. LEDs 30, 31, and 32 or photodiodes 40 and 41 of the first to fifth sensor modules 21A, 22A, 23A, 24A, and 25A according to the modified example illustrated in Fig. 5 correspond to the LEDs 30, 31, and 32 or the photodiode of the first sensor module 21 in the second embodiment and the LEDs 33 and 34 or the photodiode 42 of the sixth to eighth sensor modules according to the modified example correspond to the LEDs 33 and 34 or the photodiode 42 of the second sensor module 22 in the second embodiment.

### <Modified example of third embodiment>

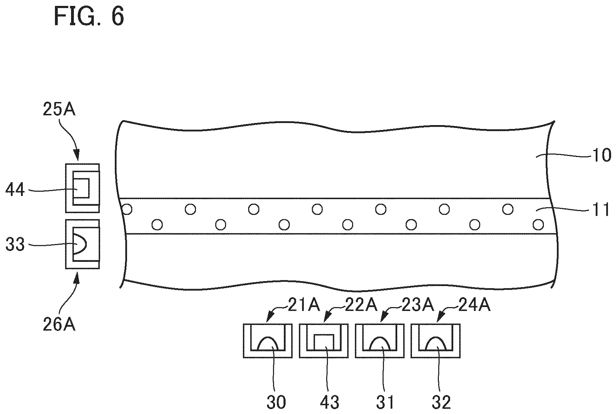

A sensor module 20A according to a modified example of the third embodiment illustrated in Fig. 6 includes first to sixth sensor modules 21A, 22A, 23A, 24A, 25A, and 26A. LEDs 30, 31, and 32 or a photodiode 43 of first to fourth sensor modules 21A, 22A, 23A, and 24A according to the modified example illustrated in Fig. 6 correspond to the LEDs 30, 31, and 32 or the photodiode 43 of the first sensor module 21 in the third embodiment and an LED 33 or a photodiode 44 of fifth and sixth sensor modules 25A and 26A according to the modified example of the third embodiment correspond to the LED 33 or the photodiode 44 of the second sensor module 22 according to the third embodiment.

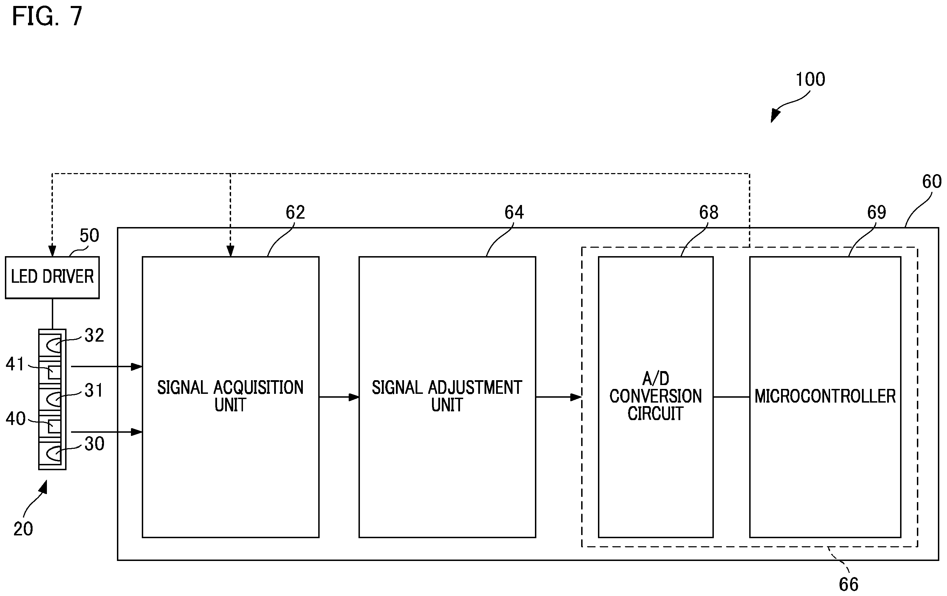

Fig. 7 is a diagram illustrating an example of a functional configuration of the biological information measurement device according to the first to third embodiments and the modified examples thereof. A biological information measurement device 100 includes a sensor module 20, an LED driver 50 connected to the sensor module, and a signal processing unit 60. The sensor module 20 will be described below with reference to the configuration of the first sensor module in the first embodiment.

The LED driver 50 is a drive circuit configured to drive LEDs 30, 31, and 32 of the sensor module 20. The LED driver 50 controls an amount of light emitted by the LEDs 30, 31, and 32 under the control of a microcontroller 69 of the signal processing unit 60. Furthermore, the LED driver 50 turns each of the LEDs 30, 31, and 32 on or off under the control of the microcontroller 69.

The signal processing unit 60 includes a signal acquisition unit 62, a signal adjustment unit 64, and a signal control unit 66. The signal acquisition unit 62 includes an amplification circuit and a sample and hold circuit. The signal adjustment unit 64 includes a low pass filter and a high pass filter configured to remove noise components. The signal control unit 66 includes an analog/digital (A/D) conversion circuit 68 and the microcontroller 69. The microcontroller 69 computes and calculates biological information and controls operations of units which constitute the biological information measurement device 100.

The LEDs 30, 31, and 32 of the sensor module 20 are selectively emitted by the LED driver 50 under the control of the microcontroller 69 and emit light having different wavelengths to an organism 10. The light reflected by the organism 10 is received by the photodiodes 40 and 41 and a signal corresponding to an amount of light received is output to the signal acquisition unit. The signal acquisition unit 62 distributes signals output from the photodiode 40 and the photodiode 41 to the amplification circuit under the control of the microcontroller 69 and outputs the signals to the signal adjustment unit 64. At this time, the sample and hold circuit of the signal acquisition unit 62 holds the signals output from the photodiodes 40 and 41 for a certain period under the control of the microcontroller 69. The low pass filter of the signal adjustment unit 64 performs low pass filtration on a signal output from the signal acquisition unit 62 at a predetermined cutoff frequency and outputs the signal to the high pass filter. The high pass filter performs high pass filtration on a signal output from the low pass filter at a predetermined cutoff frequency and outputs the signal to the signal control unit 66. Since the signal output from the high pass filter may be a weak signal to be transmitted or digitized in some cases, the signal adjustment unit 64 may further include an amplification circuit configured to amplify the signal. The A/D conversion circuit 68 of the signal control unit 66 converts a signal output from the signal adjustment unit 64 from an analog signal into a digital signal and outputs the digital signal. The microcontroller 69 performs signal processing such as waveform shaping on the basis of a digital signal output from the A/D conversion circuit 68 and generates biological information as a measurement result. The biological information to be generated includes one or more of a pulse rate, an oxygen concentration in blood, and a glucose concentration in blood. The generated biological information and signal can be stored in a memory of the microcontroller 69.

Since the biological information measurement device 100 of the present invention deals with various types of measurement of the organism 10, the microcontroller 69 can control amounts of light emitted by the LEDs 30, 31, and 32 in the LED driver 50, signal amplification in the signal acquisition unit 62, and the like in parallel on the basis of signals output from the photodiodes 40 and 41. For example, when biological tissues having a thick skin thickness are measured, relatively small amounts of light are incident on the photodiodes 40 and 41. Thus, a large amount of light is required for achieving detection of light. In this case, the microcontroller 69 can perform control, for example, so that amounts of light emitted by the LEDs 30, 31, and 32 are increased via the LED driver 50 or directly or signal amplification of the signal acquisition unit 62 is increased.

The various processes described above may be performed not only in time series in accordance with the description but also in parallel or individually in accordance with the processing capacity of the device which performs the process or necessity. Furthermore, although the embodiment in which information of the organism 10 is calculated using the microcontroller 69 has been described, biological information may be calculated through processing of a microcontroller or the like of an external device. In this case, the biological information measurement device 100 includes a communication unit which can communicate with an external device.

### <Fourth embodiment>

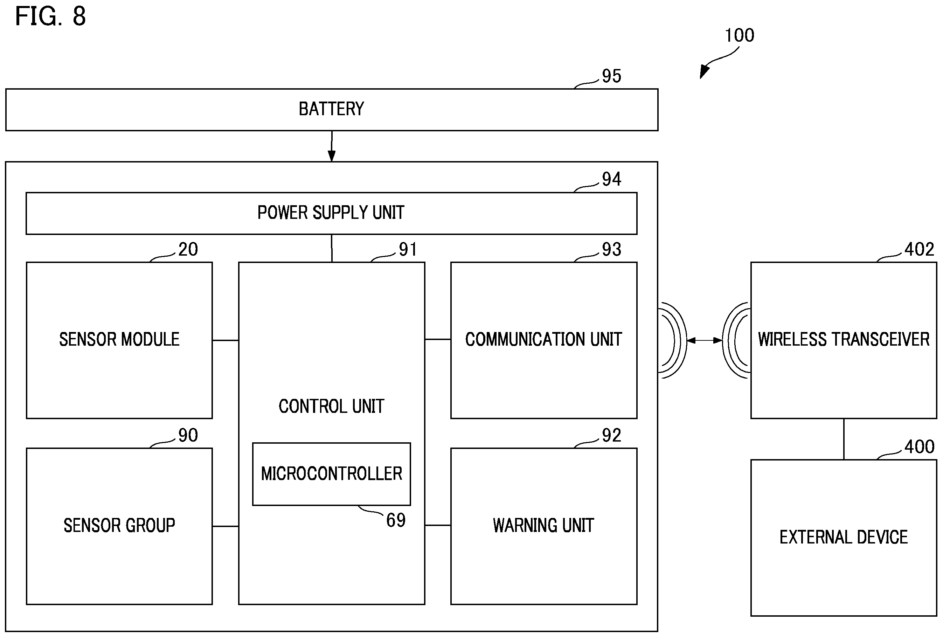

Fig. 8 is a diagram illustrating an example of a functional configuration of a biological information measurement device according to a fourth embodiment. A biological information measurement device 100 according to the fourth embodiment further includes a sensor unit 90, a control unit 91, a warning unit 92, a communication unit 93, and a power supply unit 94, in addition to the biological information measurement device 100 according to the first to third embodiments described above.

The sensor unit 90 includes a temperature sensor, a humidity sensor, an acceleration sensor, a pressure sensor, a gyro sensor, a galvanic skin response (GSR) sensor, and the like. Various sensors constituting the sensor unit are connected to the control unit 91 and output the detected signals to control unit 91.

The temperature sensor has a function of detecting an organism temperature of a subject, an external temperature, and the like. The humidity sensor has a function of detecting external humidity and the like. The acceleration sensor and the gyro sensor has a function of detecting a body movement associated with a subject's body. The biological information measurement device 100 in this embodiment may be configured to include both an acceleration sensor and a gyro sensor and use the detection results of both of the sensors together or may include only one of them. The pressure sensor has a function of detecting an external pressure such as an atmosphere pressure. The pressure sensor also has a function of detecting a pressure applied to the organism 10 and the biological information measurement device 100. A GSR sensor 86 has a function of detecting a GSR (or galvanic skin response) value and the like of a subject.

The control unit 91 also functions as the signal processing unit 60 of the biological information measurement device 100 in the first to third embodiments and is connected to the sensor module 20 and various sensors of the sensor unit 90 described above. The control unit 91 includes a microcontroller 69 and controls the overall operation of the biological information measurement device 100. The control unit 91 also has a function of storing a control program to be executed, a processing result, various data, and the like. The control unit 91 controls various sensors of the sensor module 20 and the sensor unit 90 and performs acquisition, analysis, and output operations of measurement data of biological information. These measurement data may be transmitted to an external device 400 via the communication unit 93 each time signals detected by the various sensors are acquired or may be temporarily stored in the control unit 91 and periodically transmitted. Furthermore, when the acquired measurement data is transmitted to the external device 400, the data analysis of the measurement data described above may be performed using the external device 400.

Since the signals output from the various sensors to the control unit 91 are analog signals, the control unit 91 has an A/D conversion function of converting an analog signal to be input into a digital signal. Here, when the analog signal to be input is directly transmitted to the external device 400, the A/D conversion function may be not provided.

Examples of the biological information to be obtained include sweating, a heartbeat, pulse waves, a skin temperature, a galvanic skin response, a skin resistance value, oxygen saturation in blood, a glucose concentration in blood, and the like. In addition, the biological information may be appropriately selected in accordance with the purpose of utilization of the biological information measurement device 100.

The warning unit 92 includes a light output unit which outputs predetermined light or a sound output unit which outputs predetermined sound. The warning unit 92 also has a function of emitting predetermined light or sound to a subject or the like on the basis of the detection that the acquired biological information is less than or equal to a predetermined threshold value. Furthermore, when the subject stops the measurement of biological information and then does not start the measurement for a certain period of time, the warning unit 92 may emit light or sound to prompt the subject to start the measurement again.

The communication unit 93 has a function of transmitting the measurement data which has been subjected to the signal processing using the control unit 91 to the external device 400 in a wireless or wired manner. Furthermore, the communication unit 93 has a function of transmitting a signal used for giving a notification of abnormality to the external device 400 on the basis of the detection that the acquired biological information is less than or equal to a predetermined threshold value. In this embodiment, for example, the measurement data is transmitted to the external device 400 including a wireless transceiver 402 through a method such as infrared communication or Bluetooth (registered trademark).

Also, the communication unit 93 may transmit the measurement data to the external device 400 via a cable 96. In this case, since electric power is supplied using a power supply line of this cable 96, electric power is supplied at the same time at the time of connecting to the external device 400 via the cable 96. The cable 96 may be a USB cable.

The power supply unit 94 includes a connection terminal used for connecting to an attachable/detachable external battery 95 or an external device 400 in which electric power is supplied, a power supply circuit, and a charging circuit. This connection terminal may be a USB terminal.

The external device 400 includes the wireless transceiver 402. For example, a mobile terminal, a tablet terminal, or the like may be exemplified. The external device 400 may include a display unit which can display measurement data and the like received from the communication unit 93. Thus, a subject of the biological information measurement device 100 can confirm his/her current or previous biological information using numerical values.

The biological information measurement device 100 and the external device 400 are not limited to the configuration of this embodiment and various modifications such as omission of some of these constituent elements and addition of other constituent elements are possible.

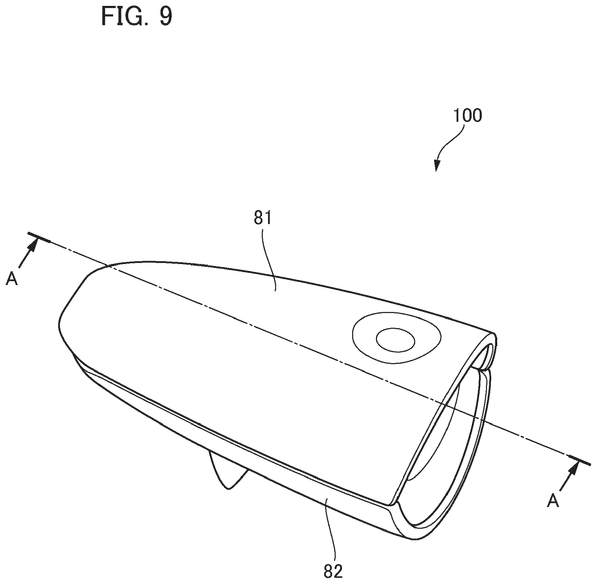

Figs. 9 to 17 are schematic diagrams of an example of an outer form of the biological information measurement device according to the fourth embodiment.

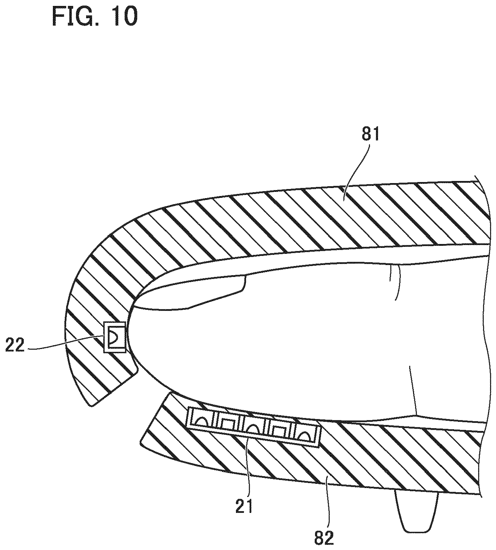

Fig. 9 is a schematic diagram of an example of a first outer form of the biological information measurement device according to the fourth embodiment. Fig. 10 is a cross-sectional view taken along line A-A of Fig. 9 when viewed in an arrow direction. A biological information measurement device 100 illustrated in Figs. 9 and 10 is a finger installation type biological information measurement device 100 in which a finger as an organism 10 is used as a target. The biological information measurement device 100 includes a housing section which includes a first housing 81 and a second housing 82 and receives the finger that is the organism 10 and a movable mechanism section (not shown) which adjusts a relative distance between the first housing 81 and the second housing 82. The first housing 81 and the second housing 82 form an accommodation section of a finger that is a measurement portion of a person to be measured between each other. The example of the outer form illustrated in Fig. 9 is a wearable type device without a wired cable.

The first sensor module 21 and the second sensor module 22 are stored in a housing. For example, the first sensor module 21 is constituted of an LED which emits red visible light of 635 to 700 nm, an LED which emits infrared light of 700 to 1000 nm, an LED which emits infrared light greater than or equal to 1000 nm, one photodiode having high detection sensitivity for wavelengths of a red visible light region and infrared light region of 700 to 1000 nm, and one photodiode having high detection sensitivity for wavelengths in an infrared light region of 700 to 1000 nm and an infrared light region greater than or equal to 1000 nm (for example, a wavelength of infrared light). The second sensor module 22 is constituted of an LED which emits infrared light greater than or equal to 1000 nm.

The positional relationship between the first sensor module 21 and the second sensor module 22 can be appropriately selected in accordance with a substance to be measured and a configuration of a device having a sensor module to be stored therein. For example, although not shown in the drawing, the first sensor module 21 and the second sensor module 22 can be provided at a position in which a tilt angle θ between an optical axis L of the LED of the second sensor module 22 and a center of a photodiode which receives light of the first sensor module 21 is larger than 0 degrees and smaller than 180 degrees. In other words, the first sensor module 21 and the second sensor module 22 are not located on the same plane of an organism 10 (the angle θ is 0 degrees) and can be provided at positions in which the first sensor module 21 and the second sensor module 22 do not face each other (the angle θ is 180 degrees) with an organism 10 therebetween.

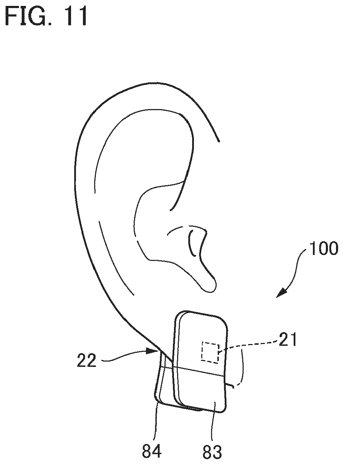

Fig. 11 is a schematic diagram of an example of a second outer form of the biological information measurement device according to the fourth embodiment. A biological information measurement device 100 illustrated in Fig. 11 has an earlobe as an organism 10 as a target. The biological information measurement device 100 illustrated in Fig. 11 has housings 83 and 84 installed to wrap an earlobe of a subject and is fixed not to fall off from the earlobe. The first sensor module 21 and the second sensor module 22 are stored in a housing. The first sensor module 21 and the second sensor module 22 are provided to be separately installed to either one of the front and back of the earlobe.

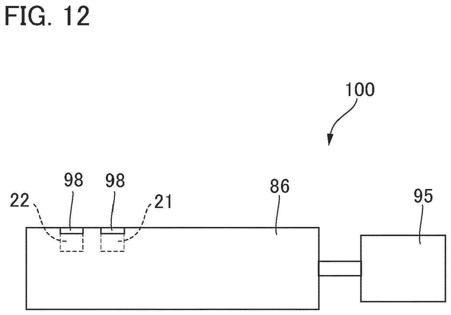

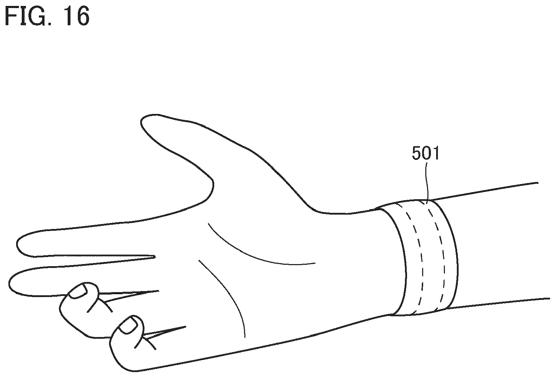

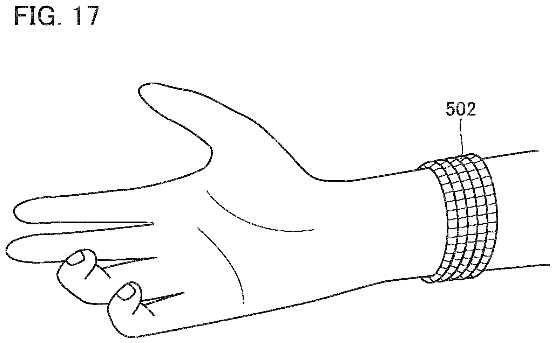

Figs. 12 to 15 are schematic diagrams of an example of third to fifth outer form of the biological information measurement device according to the fourth embodiment. Although a finger, a wrist, an arm, and the like as an organism 10, that is, a target is not particularly limited, the biological information measurement device 100 illustrated in Figs. 12 to 15 is stored inside a band section illustrated in Figs. 16 and 17 and can be used as a wrist-worn type biological information measurement device 100.

A biological information measurement device 100 illustrated in Fig. 12 includes a housing 86, a first sensor module 21 and a second sensor module 22 stored in the housing, and an attachable/detachable external battery 95. The housing 86 is a substantially rectangular parallelepiped box-shaped member. In addition, it is desirable that the housing 86 include a refracting body 98. The refracting body 98 causes an optical path of light which has been emitted from the LEDs of the first sensor module 21 and the second sensor module 22 to be refracted. Thus, even when the housing 86 does not have a bent structure, the first sensor module 21 and the second sensor module 22 can detect the light transmitted through and/or reflected by an organism 10. As the refracting body 98, a refracting body having an appropriate refractive index may be appropriately selected in accordance with an output of each LED. As the refracting body, for example, glass, a lens, or the like may be utilized.

The inside of the housing 86 serves as an installation space for various sensors and the like constituting the sensor unit 90. The biological information measurement device illustrated in Fig. 12 is a wearable type device without a wired cable. It is desirable that a thickness of the housing 86 be thin and the thickness thereof may be less than or equal to 3 mm.

Although not shown in the drawing, when the housing 86 does not include the refracting body 98, the housing 86 has a structure in which a substantially rectangular parallelepiped box-shaped member is bent so that the first sensor module 21 and the second sensor module 22 can detect the light transmitted through and/or reflected by the organism 10.

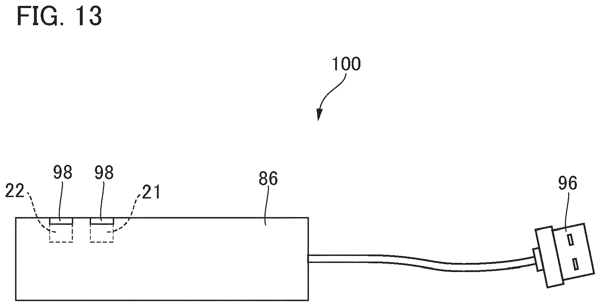

A biological information measurement device illustrated in Fig. 13 is different from the biological information measurement device illustrated in Fig. 12 in that a cable 96 is provided instead of the battery. The biological information measurement device 100 illustrated in Fig. 13 receives electric power supplied from an external device 400 through the cable 96. Furthermore, it is possible to transmit data to the external device 400 through the cable 96. The cable 96 may be a USB cable. The biological information measurement device illustrated in Fig. 13 is a portable type device.

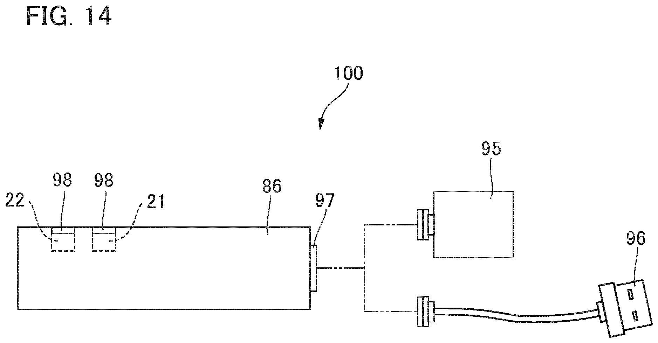

A biological information measurement device illustrated in Fig. 14 is different from the biological information measurement device illustrated in Figs. 12 and 13 in that a connection terminal 97 which can be connected to both a battery 95 and an external device 400 is provided. The connection terminal 97 may be a USB connection terminal.

A biological information measurement device illustrated in Fig. 14 may be any of the wearable type device in Fig. 12 and the portable type device in Fig. 13. According to this biological information measurement device illustrated in Fig. 14, it is not necessary to separately manufacture the wearable type device and the portable type device and it is possible to reduce the number of steps and the manufacturing costs.

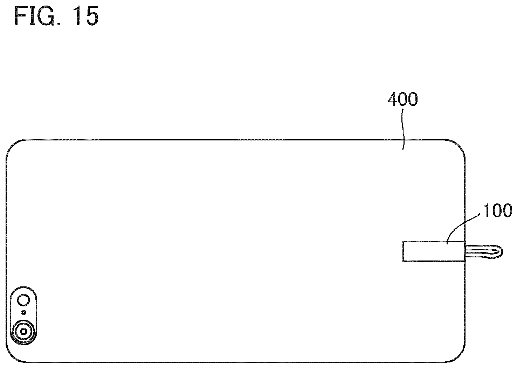

Fig. 15 is a schematic diagram of an example of a biological information measurement device to which an external device is attached. A biological information measurement device 100 is connected to an external device 400 via a cable 96 and receives electric power supplied from the external device 400 or performs data communication with the external device 400. If the external device 400 includes a display unit, a configuration in which the measurement data obtained using the biological information measurement device 100 is displayed in the external device 400 is provided. Since a decrease in size of the sensor module 20 of the present invention is realized, a thickness thereof is reduced and a size and a thickness of the housing 86 of the biological information measurement device 100 of the present invention are relatively reduced. For this reason, as shown in Fig. 15, the biological information measurement device 100 of the present invention can be provided an upper surface of the external device 400 and has excellent portability without limiting the behavior of a subject.

Figs. 16 and 17 are schematic diagrams illustrating a first band section and a second band section configured to install and fix a biological information measurement device to an organism. The band sections include a first band section 501 and a second band section 502. The first band section 501 holds the biological information measurement device 100 in an attachable/detachable manner and has an inner surface to come into contact with an organism 10. In this drawing, for example, a hole portion in which the biological information measurement device 100 can be stored is provided in a dotted line portion shown in the drawing of the first band section 501. The second band section 502 is installed above the first band section 501 and includes a stretchable light-shielding member which shields light leaking between the second band section 502 and a surface coming into contact with the organism 10 or light leaking directly.

The biological information measurement device 100 to which the present invention can be applied is not limited to the examples described above. For example, the present invention can also be applied to a wristwatch type biological information measurement device 100 in which a measurement device is stored in a wristwatch frame and an eyeglass type biological information measurement device 100 in which a measurement device is stored in a spectacle frame.

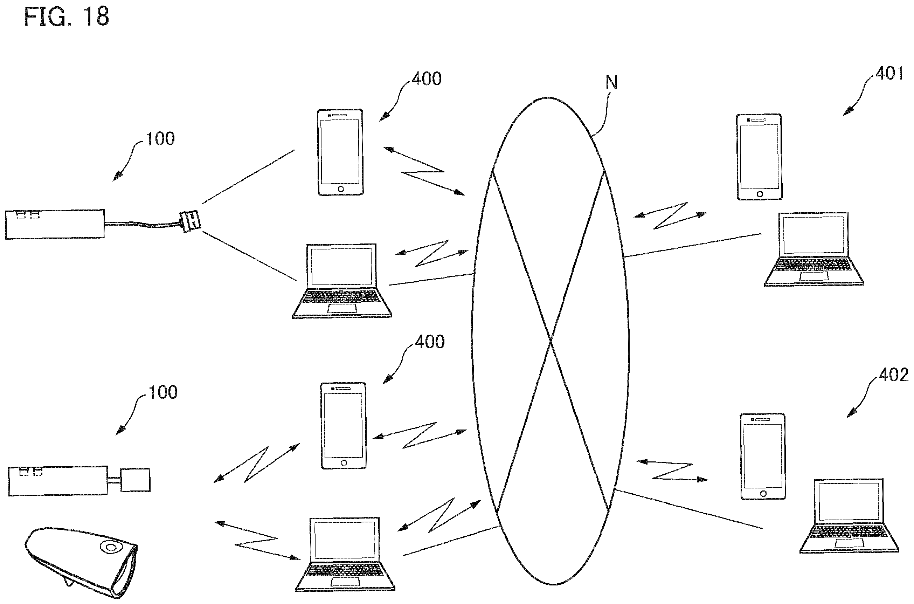

Fig. 18 is a diagram for explaining an example of a system in which the biological information measurement device of the present invention is used. If the biological information measurement device 100 of the present invention is used, when an abnormal value occurs in a biological information measurement value of a subject, it is possible to provide a warning sound and/or a warning notification screen for giving a notification of an abnormality of the subject to an external device 401 of an expert such as a doctor or an external device 402 of the subject's friend over a communication network N. The communication network N may be infrared communication, Bluetooth (registered trademark), or the like. Warning notification destinations of experts such as doctors and the subject's friends are registered in advance in the control unit of the biological information measurement device. Thus, the doctor or the subject's friend who has heard the warning sound or has seen the warning notification screen can immediately known that the biological information of the subject is abnormal and can take appropriate measures. The warning notification screen can include, for example, position information of the subject, abnormal values, information useful for coping with the subject in which the abnormality has occurred, and the like.

The above-described embodiments are merely examples of specific embodiments for carrying pout the present invention and the technical scope of the present invention need not be limitedly interpreted by there. That is to say, the present invention can be implemented in various forms without departing from the gist of the main features thereof.

Also, it is needless to say that the respective embodiments and the operation configurations described above can be implemented in any combination as long as they do not contradict each other.

Priority is claimed on Japanese Patent Application No. 2018-052385, filed March 20, 2018, the entire contents of which is incorporated herein by reference.

### [Reference Signs List]

- 10: Organism
- 11: Blood
- 21: First sensor module
- 22: Second sensor module
- 60: Signal processing unit
- 62: Signal acquisition unit
- 64: Signal adjustment unit
- 66: Signal control unit
- 68: A/D conversion circuit
- 69: Microcontroller
- 100: Biological information measurement device

## Claims

1. A biological information measurement device (100), comprising:
a first sensor module (21) which includes one or multiple light-emitting elements (30, 31, and 32) emitting light and one or multiple light-receiving elements (40 and 41) receiving light emitted from the light-emitting elements (30, 31, and 32) and outputting a signal of an intensity corresponding to an amount of light received;
a second sensor module (22) which includes one or multiple light-emitting elements emitting light (33) ; and
a signal processing unit (60) in which signal processing is performed on an output signal from the light-receiving elements (40 and 41) to generate biological information,
wherein at least one light-receiving element (40 and 41) of the first sensor module (21) receives light emitted from at least one light-emitting element (30, 31, and 32) of the first sensor module (21) and reflected by an organism (10) and receives light which has been emitted from at least one light-emitting element (33) of the second sensor module (22) and which has been transmitted through an organism (10).

2. The biological information measurement device (100) according to claim 1, wherein the number of light-emitting elements (30, 31, and 32) included in the first sensor module (21) is N (N is greater than or equal to 3),
the number of light-receiving elements (40 and 41) included in the first sensor module (21) is greater than or equal to 1 and less than or equal to N-1, and
at least one light-receiving element (40 and 41) of the first sensor module (21) receives light emitted from at least two different light-emitting elements (30, 31, and 32) of the first sensor module (21) and reflected by an organism (10).

3. The biological information measurement device (100) according to claim 1 or 2, wherein the first sensor module (21) includes:
a first light-emitting element (30) which emits light having a first wavelength;
a second light-emitting element (31) which emits light having a second wavelength;
a third light-emitting element (32) which emits light having a third wavelength;
a first light-receiving element (40) which receives light having the first wavelength and the second wavelength emitted from the first light-emitting element (30) and the second light-emitting element (31) and reflected by an organism (10) and which outputs a signal; and
a second light-receiving element (41) which receives light having the second wavelength and the third wavelength emitted from the second light-emitting element (31) and the third light-emitting element (32) and reflected by an organism (10) and which outputs a signal,
the second sensor module (22) includes: a fourth light-emitting element (33) which emits light having a fourth wavelength, and
the first light-receiving element (40) and/or the second light-receiving element (41) receives light having the fourth wavelength emitted from the fourth light-emitting element (33) and transmitted through an organism (10).

4. The biological information measurement device (100) according to claim 1 or 2, wherein the second sensor module (22) further includes one or multiple light-receiving elements (42 and 44), and
at least one light-receiving element (42 and 44) of the second sensor module (22) receives light emitted from at least one light-emitting element (30, 31, and 32) of the first sensor module (21) and transmitted through an organism (10) and receives light emitted from at least one light-emitting element (33 and 34) of the second sensor module (22) and reflected by an organism (10).

5. The biological information measurement device (100) according to claim 4, wherein the first sensor module (21) includes:
a first light-emitting element (30) which emits light having a first wavelength;
a second light-emitting element (31) which emits light having a second wavelength;
a third light-emitting element (32) which emits light having a third wavelength;
a first light-receiving element (40) which receives light having the first wavelength and the second wavelength emitted from the first light-emitting element (30) and the second light-emitting element (31) and reflected by an organism (10) and which outputs a signal; and
a second light-receiving element (41) which receives light having the second wavelength and the third wavelength emitted from the second light-emitting element (31) and the third light-emitting element (32) and reflected by an organism (10) and which outputs a signal,
the second sensor module (22) includes:
a fourth light-emitting element (33) which emits light having a fourth wavelength;
a fifth light-emitting element (34) which emits light having a fifth wavelength; and
a third light-receiving element (42) which receives light having the fourth wavelength and the fifth wavelength emitted from the fourth light-emitting element (33) and the fifth light-emitting element (34) and reflected by an organism (10) and which outputs a signal,
the first light-receiving element (40) and/or the second light-receiving element (41) receives light having the fourth wavelength and/or the fifth wavelength emitted from the fourth light-emitting element (33) and/or the fifth light-emitting element (34) and transmitted through an organism (10), and
the third light-receiving element (42) receives light having the first wavelength and/or the second wavelength and/or the third wavelength emitted from the first light-emitting element (30) and/or the second light-emitting element (31) and/or the third light-emitting element (32) and transmitted through an organism (10).

6. The biological information measurement device (100) according to claim 4, wherein the first sensor module (21) includes:
a first light-emitting element (30) which emits light having a first wavelength;
a second light-emitting element (31) which emits light having a second wavelength;
a third light-emitting element (32) which emits light having a third wavelength; and
a first light-receiving element (40) which receives light having the first wavelength, the second wavelength, and the third wavelength emitted from the first light-emitting element (30), the second light-emitting element (31), and the third light-emitting element (32) and reflected by an organism (10) and which outputs a signal,
the second sensor module (22) includes:
a fourth light-emitting element (33) which emits light having a fourth wavelength; and
a second light-receiving element (44) which receives light having the fourth wavelength emitted from the fourth light-emitting element (33) and reflected by an organism (10) and which outputs a signal,
the first light-receiving element (40) receives light having the fourth wavelength emitted from the fourth light-emitting element (33) and transmitted through an organism (10), and
the second light-receiving element (44) receives light having the first wavelength and/or the second wavelength and/or the third wavelength emitted from the first light-emitting element (30) and/or the second light-emitting element (31) and/or the third light-emitting element (32) and transmitted through an organism (10).

7. A biological information measurement device (100), comprising:
a first sensor module (21A) which includes a first light-emitting element (30) emitting light having a first wavelength;
a second sensor module (23A) which includes a second light-emitting element (31) emitting light having a second wavelength;
a third sensor module (25A) which includes a third light-emitting element (32) emitting light having a third wavelength;
a fourth sensor module (26A) which includes a fourth light-emitting element (33) emitting light having a fourth wavelength;
a fifth sensor module (22A) which includes a first light-receiving element (40) receiving light having the first wavelength and the second wavelength emitted from the first light-emitting element (30) and the second light-emitting element (31) and reflected by an organism (10) and outputting a signal;
a sixth sensor module (24A) which includes a second light-receiving element (41) receiving light having the second wavelength and the third wavelength emitted from the second light-emitting element (31) and the third light-emitting element (32) and reflected by an organism (10) and outputting a signal; and
a signal processing unit (60) in which signal processing is performed on output signals from the first light-receiving element (40) and the second light-receiving element (41) to generate biological information,
wherein the first light-receiving element (40) and/or the second light-receiving element (41) receives light having the fourth wavelength emitted from the fourth light-emitting element (33) and transmitted through an organism (10).

8. The biological information measurement device (100) according to claim 7, further comprising:
a seventh sensor module (28A) which includes a fifth light-emitting element (34) emitting light having a fifth wavelength; and
an eighth sensor module (27A) which includes a third light-receiving element (42) receiving light having the fourth wavelength and the fifth wavelength emitted from the fourth light-emitting element (33) and the fifth light-emitting element (34) and reflected by an organism (10) and outputting a signal,
wherein the first light-receiving element (40) and/or the second light-receiving element (41) receives light having the fourth wavelength and/or the fifth wavelength emitted from the fourth light-emitting element (33) and/or the fifth light-emitting element (34) and transmitted through an organism (10) and outputs a signal,
the third light-receiving element (42) receives light having the first wavelength and/or the second wavelength and/or the third wavelength emitted from the first light-emitting element (30) and/or the second light-emitting element (31) and/or the third light-emitting element (32) and transmitted through an organism (10) and outputs a signal, and
the signal processing unit (60) performs signal processing on output signals from the first light-receiving element (40), the second light-receiving element (41), and the third light-receiving element (42) to generate biological information.

9. A biological information measurement device (100), comprising:
a first sensor module (21A) which includes a first light-emitting element (30) emitting light having a first wavelength;
a second sensor module (23A) which includes a second light-emitting element (31) emitting light having a second wavelength;
a third sensor module (24A) which includes a third light-emitting element (32) emitting light having a third wavelength;
a fourth sensor module (26A) which includes a fourth light-emitting element (33) emitting light having a fourth wavelength;
a fifth sensor module (22A) which includes a first light-receiving element (43) receiving light having the first wavelength, the second wavelength, and the third wavelength emitted from the first light-emitting element (30), the second light-emitting element (31), and the third light-emitting element (32) and reflected by an organism (10) and outputting a signal;
a sixth sensor module (25A) which includes a second light-receiving element (44) receiving light having the fourth wavelength emitted from the fourth light-emitting element (33) and reflected by an organism (10) and outputting a signal; and
a signal processing unit (60) in which signal processing is performed on output signals from the first light-receiving element (40) and the second light-receiving element (41) to generate biological information,
wherein the first light-receiving element (43) receives light having the fourth wavelength emitted from the fourth light-emitting element (33) and transmitted through an organism (10), and
the second light-receiving element (44) receives light having the first wavelength and/or the second wavelength and/or the third wavelength emitted from the first light-emitting element (30) and/or the second light-emitting element (31) and/or the third light-emitting element (32) and transmitted through an organism (10).

10. The biological information measurement device (100) according to claims 3, 6, 7, and 9, wherein the first wavelength is a wavelength in an infrared light region of greater than or equal to 1000 nm,
the second wavelength is a wavelength in an infrared light region of less than or equal to 1000 nm,
the third wavelength is a wavelength in a visible light region of greater than or equal to 390 nm, and
the fourth wavelength is a wavelength in an infrared light region of greater than or equal to 1000 nm.

11. The biological information measurement device (100) according to claim 5 or 8, wherein the first wavelength is a wavelength in an infrared light region of greater than or equal to 1000 nm,
the second wavelength is a wavelength in an infrared light region of less than or equal to 1000 nm,
the third wavelength is a wavelength in a visible light region of greater than or equal to 390 nm,
the fourth wavelength is a wavelength in an infrared light region of greater than or equal to 1000 nm, and
the fifth wavelength is a wavelength in a visible light region of greater than or equal to 390 nm.

12. The biological information measurement device (100) according to any one of claims 1 to 11, wherein the biological information includes one or more of a pulse rate, an oxygen concentration in blood, and a glucose concentration in blood.

13. The biological information measurement device (100) according to any one of claims 1 to 12, further comprising:
a sensor unit which includes one or more of a temperature sensor configured to detect a temperature of the organism (10) or an outside temperature, a humidity sensor configured to detect an external humidity, a pressure sensor configured to detect an external pressure, an acceleration sensor configured to detect the movement associated with the organism (10), a gyro sensor, and an galvanic skin response (GSR) sensor configured to detect a GSR of the organism (10);
a communication unit (93) which communicates with an external device (400) ; and
a control unit (91) which causes the communication unit (93) to transmit a detection value obtained by the sensor unit to the external device (400) together with the biological information.

14. The biological information measurement device (100) according to claim 13, wherein the control unit (91) detects an abnormality by comparing the biological information with a threshold value, and
the biological information measurement device includes a warning unit (92) which outputs light or a sound or which transmits a notification signal concerning the abnormality to the external device (400) via the communication unit (93) if the control unit (91) detects an abnormality.

15. The biological information measurement device (100) according to claim 13 to 14, wherein the organism (10) which is an object on which measurement is to be performed is a finger.
